# EUROPEAN PATENT APPLICATION

(11) **EP 2 899 270 A1**
(43) Date of publication of application: **29.07.2015**
(21) Application number: 13838126.4
(22) Date of filing: 20.08.2013
(51) Int. Cl.: C12N 15/09, C12P 7/18

(54) **METHOD FOR PRODUCING BUTANEDIOL**

(30) Priority: 24.09.2012 JP 2012209981
(71) Applicant: Showa Denko K.K., Tokyo 105-8518 (JP)
(72) Inventor: AOKI, Hirobumi, Tokyo 105-8518 (JP); KOKIDO, Yuzuru, Tokyo 105-8518 (JP); YONEDA, Tadashi, Tokyo 105-8518 (JP)
(74) Representative: Strehl Schübel-Hopf & Partner
(86) International application number: PCT/JP2013/072186
(87) International publication number: WO 2014/045781

(57) **Abstract**

A manufacturing method for a butanediol includes a step that cultures a microbe that contains a gene that codes acetoacetyl-CoA synthase that catalyzes a reaction that irreversibly produces acetoacetyl-CoA from acetyl-CoA and malonyl-CoA, acetoacetyl-CoA reductase that catalyzes a reaction that produces 3-hydroxybutyryl-CoA from acetoacetyl-CoA, and an enzyme that catalyzes a reaction for producing a butanediol from 3-hydroxybutyryl-CoA.

## Description

### TECHNICAL FIELD

The present invention relates to a manufacturing method for a butanediol.

### BACKGROUND ART

In recent years, attention is paid to a compound manufacturing process with a renewable source as a raw material from the viewpoint of depletion of fossil resource, a countermeasure for global warming, or the like. In particular, a so-called bio-refinery has widely been studied wherein a variety of compounds as raw materials for a polymer or compounds as raw materials for a chemical product are manufactured in biochemical processes with biomass as raw materials.

For compounds that are expected as raw material conversion of biomass, there are provided butanediols (that refer to 1,3-butanediol and 1,4-butanediol). For example, 1,4-butanediol is used as a synthetic raw material for an organic chemical product, a monomer unit of a polyester and an engineering plastic, or the like, and 1,3-butanediol is used as an organic solvent, a substrate for cosmetic product, or the like. Demand of these butanediols is high and a need for a manufacturing method for a butanediol by a biochemical process with a renewable source such as biomass as a raw material is increased.

For manufacturing methods for a butanediol that use a biochemical process, there are provided, for example, methods described in patent documents 1 to 3 and non-patent document 1.

### PRIOR ART DOCUMENTS

[Patent Document 1] Japanese Patent No. 4380704 specification
[Patent Document 2] International Publication No. 2008/115840 official gazette
[Patent Document 3] International Publication No. 2010/127319 official gazette
[Non-patent document 1] Harry Yim et al., Metabolic engineering of Escherichia coli for direct production of 1,4-butanediol, Nature Chemical Biology, 7, 445-452 (2011).

### DISCLOSURE OF THE INVENTION

### PROBLEMS TO BE SOLVED BY THE INVENTION

However, methods described in patent documents 1 to 3 and non-patent document 1 are complicated processes.

Against a problem as described above, there is provided a new manufacturing method for a butanediol that is able to obtain a butanediol economically.

### MEANS FOR SOLVING THE PROBLEM

The present invention included the following.
[1] A manufacturing method for a butanediol, including a step that cultures a microbe that contains a gene that codes acetoacetyl-CoA synthase that catalyzes a reaction that irreversibly produces acetoacetyl-CoA from acetyl-CoA and malonyl-CoA, acetoacetyl-CoA reductase that catalyzes a reaction that produces 3-hydroxybutyryl-CoA from acetoacetyl-CoA, and an enzyme that catalyzes a reaction for producing a butanediol from 3-hydroxybutyryl-CoA.
[2] The manufacturing method for a butanediol as described in [1], wherein the enzyme that catalyzes a reaction for producing a butanediol from 3-hydroxybutyryl-CoA is enoyl-CoA hydratase that catalyzes a reaction that produces crotonyl-CoA from 3-hydroxybutyryl-CoA, 4-hydroxybutyryl-CoA dehydratase that catalyzes a reaction that produces 4-hydroxybutyryl-CoA from crotonyl-CoA, and an aldehyde dehydrogenase that catalyzes a reaction that produces 4-hydroxybutanal from 4-hydroxybutyryl-CoA or an alcohol dehydrogenase that catalyzes a reaction that produces 1,4-butanediol therefrom.
[3] The manufacturing method for a butanediol as described in [1], wherein the enzyme that catalyzes a reaction for producing a butanediol from 3-hydroxybutyryl-CoA is an aldehyde dehydrogenase that catalyzes a reaction that produces 3-hydroxybutanal from 3-hydroxybutyryl-CoA or an alcohol dehydrogenase that catalyzes a reaction that produces 1,3-butanediol therefrom.
[4] The manufacturing method for a butanediol as described in any of [1] to [3], wherein a gene that codes acetoacetyl-CoA synthase that catalyzes a reaction that irreversibly produces acetoacetyl-CoA from acetyl-CoA and malonyl-CoA is a gene described in any of undermentioned (a) - (c).
   (a) a gene that has a base sequence of sequence number 1
   (b) a gene that has a base sequence such that one or more bases are deleted, substituted, or added in a base sequence of sequence number 1, wherein the gene has a base sequence with an identity greater than or equal to 90% with respect to the base sequence of sequence number 1
   (c) a gene that hybridizes with a gene that has a base sequence complimentary with a gene that has a base sequence described in sequence number 1 on a stringent condition
[5] The manufacturing method for a butanediol as described in [1], [2], or [4], wherein the microbe contains a gene that codes 4-hydroxybutyryl-CoA dehydratase described in any of undermentioned (d) - (f).
   (d) a gene that has a base sequence of sequence number 8
   (e) a gene that has a base sequence such that one or more bases are deleted, substituted, or added in a base sequence of sequence number 8, wherein the gene has a base sequence with an identity greater than or equal to 90% with respect to the base sequence of sequence number 8
   (f) a gene that hybridizes with a gene that has a base sequence complimentary with a gene that has a base sequence described in sequence number 8 on a stringent condition
[6] The manufacturing method for a butanediol as described in any of [1] to [5], wherein the microbe further contains a gene that codes acetyl-CoA carboxylase that catalyzes a reaction that produces malonyl-CoA wherein acetyl-CoA is a substrate.
[7] The manufacturing method for a butanediol as described in any of [1] to [6], wherein the step that cultures includes a step that cultures the microbe on an aerobic condition.
[8] The manufacturing method for a butanediol as described in any of [1] to [7], wherein the microbe is Escherichia coli, a yeast, a corynebacterium, or a clostridium.

### EFFECTS OF THE INVENTION

It is possible to provide a manufacturing method for a butanediol that is able to obtain a butanediol economically.

### BRIEF DESCRIPTION OF THE DRAWINGS

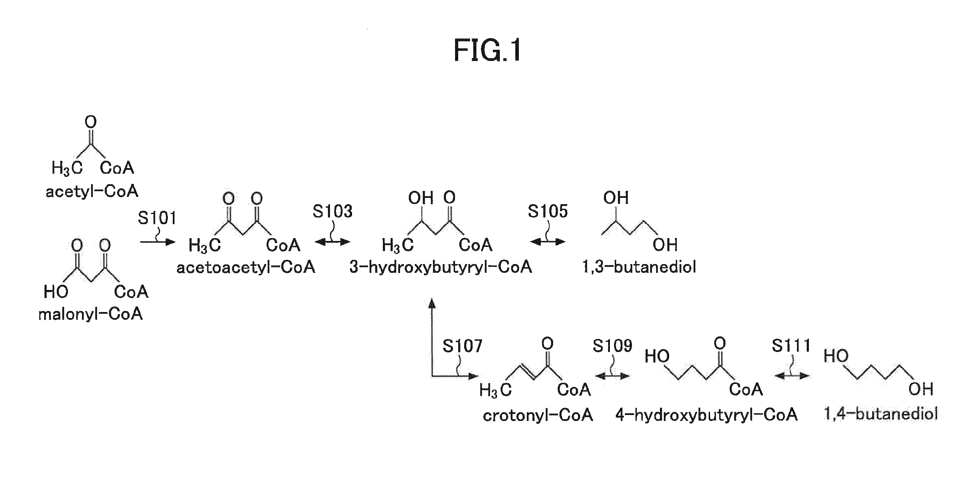

[FIG. 1] is a schematic diagram for illustrating an example of a manufacturing method for a butanediol according to the present embodiment.

### BEST MODE FOR IMPLEMENTING THE INVENTION

The present invention will be described in detail below. Here, "CoA" means coenzyme A in the present specification. Furthermore, "%" indicates % by mass unless otherwise described.

FIG. 1 illustrates a schematic diagram for illustrating an example of a manufacturing method for a butanediol according to the present embodiment.

In the present embodiment, a gene that codes acetoacetyl-CoA synthase that catalyzes a reaction (S101) that irreversibly produces acetoacetyl-CoA from acetyl-CoA and malonyl-CoA, acetoacetyl-CoA reductase that catalyzes a reaction (S103) that produces 3-hydroxybutyryl-CoA from acetoacetyl-CoA, and an enzyme that catalyzes a reaction for producing a butanediol from 3-hydroxybutyryl-CoA is expressed in a microbial body by means of transformation or the like and acetyl-CoA is supplied into such a microbial body, so that it is possible to obtain a butanediol. Here, "supply" herein also includes providing a condition that it is possible to synthesize a substance in a microbial body, other than providing such a substance from exterior. Furthermore, in a latter case, a genetic system that is intrinsically possessed by a microbe may be utilized and a genetic system may newly be introduced to be able to synthesize (similar, below).

Herein, for example, a reaction for producing a butanediol from 3-hydroxybutyryl-CoA may be to convert 3-hydrpxybutyryl-CoA into a butanediol at a single step as illustrated by S105 in FIG. 1 or may be to execute such conversion at multiple steps as illustrated by S107, S109, and S111 in FIG. 1.

In a case where a reaction from 3-hydroxybutyryl-CoA to a butanediol is of a single step, for example, a gene that codes acetoacetyl-CoA synthase that catalyzes a reaction that irreversibly produces acetoacetyl-CoA from acetyl-CoA and malonyl-CoA, acetoacetyl-CoA reductase that catalyzes a reaction that produces 3-hydroxybutyryl-CoA from acetoacetyl-CoA, and an aldehyde dehydrogenase that catalyzes a reaction that produces 3-hydroxybutanal from 3-hydroxybutyryl-CoA or an alcohol dehydrogenase that catalyzes a reaction that produces 1,3-butanediol therefrom is expressed in a microbial body by means of transformation or the like and acetyl-CoA and malonyl-CoA are supplied into such a microbial body, so that it is possible to obtain a butanediol. In a case where an aldehyde dehydrogenase is used that produces 3-hydroxybutanal, 3-hydroxybutanal to be produced may be introduced into a butanediol due to action of an alcohol dehydrogenase that is possessed by a host microbe or may be expressed in a microbial body in combination with an alcohol dehydrogenase that introduces 3-hydroxybutanal into a butanediol.

As acetyl-CoA and malonyl-CoA are supplied into a microbial body as described below, acetoacetyl-CoA is irreversibly produced from acetyl-CoA and malonyl-CoA (S101), 3-hydroxybutyryl-CoA is produced from produced acetoacetyl-CoA (S103), and 1,3-butanediol is produced from produced 3-hydroxybutyryl-CoA (S105), due to action of a gene as described above.

In a case where a reaction from 3-hydroxybutyryl-CoA to a butanediol is of multiple steps, for example, a gene that codes acetoacetyl-CoA synthase that catalyzes a reaction that irreversibly produces acetoacetyl-CoA from acetyl-CoA and malonyl-CoA, acetoacetyl-CoA reductase that catalyzes a reaction that produces 3-hydroxybutyryl-CoA from acetoacetyl-CoA, 3-hydroxybutyryl-CoA dehydratase that catalyzes a reaction that produces crotonyl-CoA from 3-hydroxybutyryl-CoA, 4-hydroxybutyryl-CoA dehydratase that catalyzes a reaction that produces 4-hydroxybutyryl-CoA from crotonyl-CoA, and an aldehyde dehydrogenase that catalyzes a reaction that produces 4-hydroxybutanal from 4-hydroxybutyryl-CoA or an alcohol dehydrogenase that catalyzes a reaction that produces 1,4-butanediol therefrom is expressed in a microbial body by means of transformation or the like and acetyl-CoA and malonyl-CoA are supplied into such a microbial body so that it is possible to obtain a butanediol. In a case where an aldehyde dehydrogenase is used that produces 4-hydroxybutanal, 4-hydroxybutanal to be produced may be introduced into a butanediol due to action of an alcohol dehydrogenase that is possessed by a host microbe or may be expressed in a microbial body in combination with an alcohol dehydrogenase that introduces 4-hydroxybutanal into a butanediol.

As illustrated in FIG. 1, as acetyl-CoA and malonyl-CoA are supplied into a microbial body as described below, acetoacetyl-CoA is irreversibly produced from acetyl-CoA and malonyl-CoA (S101), 3-hydroxybutyryl-CoA is produced from produced acetoacetyl-CoA (S103), crotonyl-CoA is produced from produced 3-hydroxybutyryl-CoA (S107), 4-hydroxybutyryl-CoA is produced from produced crotonyl-CoA (S109), and 1,4-butanediol is produced from produced 4-hydroxybutyryl-CoA (S111), due to action of a gene as described above.

Next, an enzyme that catalyzes each reaction and a gene that codes such an enzyme will be described in detail.

### (A gene that codes actoacetyl-CoA synthase)

A butanediol that is obtained in the present embodiment is derived from acetoacetyl-CoA by using a microbe that contains a gene that codes acetoacetyl-CoA synthase that catalyzes a reaction that irreversibly produces acetoacetyl-CoA from acetyl-CoA and malonyl-CoA.

It is possible to arbitrarily select acetoacetyl-CoA synthase that is able to be used in the present embodiment as long as a reaction that irreversibly produces acetoacetyl-CoA is catalyzed wherein acetyl-CoA and malonyl-CoA are substrates. For a specific example, there is provided, for example, acetyl-CoA: malonyl-CoA acyltransferase described in "Unprecedented acetoacetyl-coenzyme A synthesizing enzyme of the thiolase superfamily involved in the mevalonate pathway., Proc. Natl. Acad. Sci., U. S. A. 107, 11265 - 11270 (2010)" (Enzyme Commission number (EC number): 2. 3. 1. 194) or a homolog thereof.

For a base sequence of a gene that codes caetoacetyl-CoA synthase as described above, it is possible to refer to accession No. AB540131 of DNA Data Bank of Japan (DDBJ).

For an enzyme that produces actoacetyl-CoA, β-ketothiolase has been known conventionally. β-ketothiolase is an enzyme that catalyzes a reaction that produces acetoacetyl-CoA wherein two molecules of acety-CoA are substrates, and is coded by a gene such as thiL or phaA. However, β-ketothiolase is an enzyme that reversibly catalyzes a reaction that synthesizes acetoacetyl-CoA as described previously and a reaction that hydrolyzes acetoacetyl-CoA to produce two molecules of acetyl-CoA. Because acetoacetyl-CoA synthase in the present embodiment irreversibly catalyzes a reaction that synthesizes acetoacetyl-CoA, a yield of acetoacetyl-CoA is high in a manufacturing method that uses such an enzyme in the present embodiment.

Furthermore, a gene that codes acetyl-CoA carboxylase that catalyzes a reaction that produces malonyl-CoA wherein acetyl-CoA is a substrate may be used in the present embodiment. Because supply of malonyl-CoA is increased by using acetyl-CoA carboxylase, an irreversible reaction for acetoacetyl-CoA as described previously is accelerated. Because it is possible to increase carbon flux to a product accordingly, it is possible to manufacture a butanediol more efficiently. Here, acetyl-CoA in the present embodiment is derived from an arbitrary carbon source to be used for culturing, for example, a sugar such as glucose and is supplied from a glycolysis system that is possessed by a host.

Acetyl-CoA carboxylase that is used in the present embodiment is not particularly limited, as long as an enzyme catalyzes a reaction that produces malonyl-CoA wherein acetyl-CoA is a substrate. For a specific example, there is provided acetyl-CoA carboxylase originating from Corynebacterium (EC number: 6. 4. 1. 2) as described in a non-patent document of "Efficient production of (2S)-flavanones by Escherichia coli containing an artificial biosynthetic gene cluster, Applied Microbiology and Biotechnology, 68 498-504 (2005)" or a homolog thereof. It is known that an activity of acetyl-CoA carboxylase originating from Corynebacterium is expressed by a plurality of subunits. For one example of a base sequence of a gene that codes acetyl-CoA carboxylase, it is possible to refer to accession Nos. YP224999 and CCH23890 of DDBJ.

### (A gene that codes acetoacetyl-CoA reductase)

Acetoacetyl-CoA reductase that is used in the present embodiment is not particularly limited as long as an enzyme catalyzes a reaction that reduces actoacetyl-CoA to produce 3-hydroxybutyryl-CoA. For a specific example, there is provided acetoacetyl-CoA reductase (EC number: 1. 1. 1. 36), 3-hydroxybutyryl-CoA dehydrogenase (EC number: 1. 1. 1. 35), 3-hydroxyacyl-CoA dehydrogenase (EC number: 1. 1. 1. 157), a homolog thereof, or the like. For example, a detail of acetoacetyl-CoA reductase (EC number: 1. 1. 1. 36) and a gene thereof is described in "Poly-beta-hydroxybutyrate biosynthesis in Alcaligenes eutrophus H16. Characterization of the genes encoding beta-ketothiolase and acetoacetyl-CoA reductase., The Journal of Biological Chemistry, 264, 15293-15297 (1989)".

Here, it is possible to obtain (R)-3-hydroxybutyryl-CoA in a case where acetoacetyl-CoA reductase (EC number: 1. 1. 1. 36) is used, or it is possible to obtain (S)-3-hydroxybutyryl-CoA in a case where 3-hydroxybutyryl-CoA dehydrogenase (EC number: 1. 1. 1. 35) or 3-hydroxyacyl-CoA dehydrogenase (EC number: 1. 1. 1. 157) is used. In the present embodiment, it is possible to selectively use an enzyme as described above, depending on a desired chirality in manufacturing of 1,3-butanediol.

### [A gene that codes enoyl-CoA hydratase]

Enoyl-CoA hydratase that is used in the present embodiment is not particularly limited as long as an enzyme catalyzes a reaction that dehydrates 3-hydroxybutyryl-CoA to produce crotonyl-CoA. For a specific example of an enzyme that catalyzes a reaction that produces crotonyl-CoA from (S)-3-hydroxybutyryl-CoA, there is provided enoyl-CoA hydratase (EC number: 4. 2. 1. 17), a homolog thereof, or the like. For a detail of a gene that codes enoyl-CoA hydratase (EC number: 4. 2. 1. 17), it is possible to refer to a non-patent document of "The complete stereochemistry of the enzymatic dehydration of 4-hydroxybutyryl coenzyme A to crotonyl coenzyme. A., Friedrich P, Darley DJ, Golding BT, Buckel W., Angewandte Chemie International Edition, 2008 47 (17) 3254 - 3257 (2008)" or the like. Furthermore, for an enzyme that catalyzes a reaction that produces crotonyl-CoA from (R)-3-hydroxybutyryl-CoA, there is provided enoyl-CoA hydratase (EC number: 4. 2. 1. 119), a homolog thereof, or the like. For a detail of a gene that codes enoyl-CoA hydratase (EC number: 4. 2. 1. 119), it is possible to refer to a non-patent document of "Metabolism of poly-beta-hydroxybutyrate. II. Enzymatic synthesis of D-(-)-beta hydroxybutyryl coenzyme A by an enoyl hydrase from Rhodospirillum rubrum., Moskowitz GJ, Merrick JM. Journal Biochemistry., 8 2748 - 2755 (1969)" or the like.

As described previously, it is possible for acetoacetyl-CoA reductase that catalyzes a reaction that produces 3-hydroxybutyryl-CoA from acetoacetyl-CoA to produce an (R)-body and/or (S)-body of 3-hydroxybutyryl-CoA depending on a kind of an enzyme to be applied. Furthermore, enoyl-CoA hydratase that catalyzes a reaction that produces crotonyl-CoA from 3-hydroxybutyryl-CoA also has a similar selectivity of an optical isomer. That is, it is possible for a manufacturing method for a butanediol according to the present embodiment to combine optical selectivities of acetoacetyl-CoA reductase and enoyl-CoA hydratase suitably, and it is possible to control a reaction that produces crotonyl-CoA from acetoacetyl-CoA.

### (A gene that codes 4-hydroxybutyryl-CoA dehydrogenase)

For a gene that codes an enzyme that catalyzes a reaction that produces 4-hydroxybutyryl-CoA from crotonyl-CoA in the present embodiment, for example, a gene indicated by sequence number 8 is used. In the present embodiment, a homolog of a gene indicated by sequence number 8 may be provided as long as a gene codes an enzyme that catalyzes a reaction that produces 4-hydorxybutyryl-CoA from crotonyl-CoA, and preferably, a gene that has a base sequence described in sequence number 8 or a base sequence wherein one or several bases thereof are deleted, substituted or added is provided.

A result of translation of a gene indicated by sequence number 8 into an amino acid sequence was homologous with a result of translation of an abfD gene originating from Clostridium aminobutyricum that is able to be referred to by accession No. AJ250267 of GenBank.

A detail of an enzyme that is coded by an abfD gene is described in a non-patent document of "Fermentation of 4-aminobutyrate by Clostridium aminobutyricum: cloning of two genes involved in the formation and dehydration of 4-hydroxybutyryl-CoA, Archives of Microbiology, 174(3) 189 - 199 (2000)" or the like. It is considered that a gene indicated by sequence number 8 codes an enzyme that has a 4-hydroxybutyryl-CoA activity, based on a reaction a reaction provided by a result of translation and a gene as described previously. However, in a case where a recombinant that contains an abfD gene sequence was used as a comparative example, it was not possible to manufacture a butanediol. That is, it is considered that it is possible to manufacture 1,4-butanediol by co-expression of a gene that has a base sequence of sequence number 8 and (an)other gene(s) in a recombinant in the present embodiment. Here, amino acid sequences that code a gene indicated by sequence number 8 in the present embodiment and an abfD gene as describe previously are homologous, but a homology of base sequences wherein gene information processing software (GENETYX; produced by GENETYX CORPORATION) is used is 75% and greatly different. In the present embodiment wherein genes with base sequences that are greatly different are applied, it is possible to provide an efficient manufacturing method for a butanediol.

### (A gene that codes an aldehyde dehydrogenase or an alcohol dehydrogenase)

An aldehyde dehydrogenase that is used in the present embodiment is not particularly limited as long as an enzyme catalyzes a reaction that reduces hydroxybutyryl-CoA to produce hydroxylbutanal. Here, produced hydroxylbutanal is further reduced by an alcohol dehydrogenase originating from a host microbe to produce a butanediol. Furthermore, it is possible to select a substrate(s) that is/are 3-hydroxybutyryl-CoA and/or 4-hydroxybutyryl-CoA, depending on a butanediol that is a target. For one example of an aldehyde hydrogenase, there is provided aldehyde dehydrogenase (EC number: 1. 2. 1. 10) of Clostridium beijerinckii strain NRRL B592 strain or a homolog thereof. It is possible to refer to a base sequence of a gene that codes aldehyde dehydrogenase (EC number: 1. 2. 1. 10) by accession No. AY20821 of DDBJ.

An alcohol dehydrogenase that is used in the present embodiment is not particularly limited as long as an enzyme catalyzes a reaction that reduces hydroxybutyryl-CoA to produce a butanediol. It is possible to select a substrate(s) that is/are 3-hydroxybutyryl-CoA and/or 4-hydroxybutyryl-CoA, depending on a butanediol that is a target. For one example of an alcohol dehydrogenase, there is provided alcohol dehydrogenase (EC number: 1. 1. 1. 1) of Clostridium acetobutylicum ATCC 824 strain or a homolog thereof. For a base sequence of a gene that codes alcohol dehydrogenase (EC number: 1. 1. 1. 1), it is possible to refer to Gene ID No. 1116040 of National Center for Biotechnology Information (NCBI).

In a preferred mode of a manufacturing method for a butanediol in the present embodiment, a series of enzyme groups that are selected depending on a desired butanediol are co-expressed in a microbial body transformed by means of gene recombination. For example, for manufacturing 1,3-butandiol, a gene that codes acetoacetyl-CoA synthase, 3-hydroxybutyryl-CoA dehydrogenase, an aldehyde dehydrogenase or an alcohol dehydrogenase, or a homolog thereof, is inserted into an arbitrary vector separately or as a serial cluster, and a host microbe is transformed. An obtained transformed body is cultured in a medium with a carbon source that is, for example, glucose, and thereby, each gene is expressed. In a case of a gene that is able to configured and expressed in a host, a transformed body is cultured in a culture medium so that such a gene is expressed. On the other hand, in a case where each gene is configured under a control of a regulator arranged on a vector, an inductive substrate is added to transfer to inductive environment, and thereby, each coding gene is expressed. While acetyl-CoA that is supplied from a glycolysis system of a host is a reaction raw material, a reaction proceeds so that a butanediol such as 1,3-butanediol is manufactured efficiently. Here, culturing in the present embodiment includes all of culturing conditions for normal microbe culturing, and further, a culturing step means that a microbe is cultured for a period of time and on a condition enough to manufacture a butanediol.

A gene that codes each enzyme that is used in the present embodiment may be configured to directly use a gene that has a base sequence originating from an original microbe. Furthermore, in a case where a host microbe is used that is different from a microbe with a gene originating therefrom, a gene with a substituted part of a base sequence may be used in order to provide good gene expression in such a host microbe. In this case, first, an original base sequence registered in a publicly known data base is used to determine a base sequence to be substituted by a person(s) skilled in the art depending on a frequency of utilization of a codon and/or a GC content. A gene with a substituted part of base sequence determined by a person(s) skilled in the art is chemically synthesized and thereby obtained.

For an example of a gene that codes each enzyme, there is provided, for example, a gene of acetoacetyl-CoA synthase (NphT7) originating from a Streptomyces genus (Streptomyces) microbe, a gene of acetoacetyl-CoA reductase (PhaB) originating from a Ralstonia genus (Ralstonia) microbe, a gene of aldehyde dehydrogenase (Ald) and/or a gene of alcohol dehydrogenase (AdhE) that originate from a Crostridium genus (Crostridium) microbe, a gene of acetyl-CoA carboxylase (Acc) originating from a Corynebacterium genus microbe, or a gene of 3-hydroxybutyryl-CoA dehydratase (Crt) originating from a Crostridium genus (Crostridium) microbe.

Furthermore, as described previously, it is possible to use a gene indicated by sequence number 8 as a gene that codes an enzyme that catalyzes a reaction that produces 4-hydroxybutyryl-CoA from crotonyl-CoA. A gene indicated by sequence number 8 is homologous with a result of transformation of a gene (abfD) that codes 4-hydroxybutyryl-CoA dehydratase.

### (A microbe)

An example of a transformation host microbe that is used in the present embodiment is not particularly limited as long as it is possible to apply a gene recombination technique to such a microbe. From the viewpoint of industrial availability, there is provided Escherichia coli, a yeast, a coryneform bacteria, or a clostridial bacteria, as a specific example. For a yeast, there is provided Saccharomyces cerevisiae, Schizosaccharomyces pombe, Kluyveromyces lactis, Kluyveromyces marxianus, or the like. For a coryneform bacteria, there is provided, Corynebacterium glutamicum, Corynebacterium efficiens, Brevibacterium divaricatum, Brevibacterium saccharolyticum, Brevibacterium immariophilum, Brevibacterium lactofermentum, Brevibacterium roseum, Brevibacterium flavum, Brevibacterium thiogenitalis, Corynebacterium acetoacidophilum, Corynebacterium acetoglutamicum, Corynebacterium callunae, Corynebacterium lilium, Corynebacterium mellassecola, Microbacterium ammoniaphilum, or the like. For a clostridial bacteria, there is provided Clostridium kluyveri, Clostridium acetobutylicum, Clostridium aminobutyricum, Clostridium beijerinckii, Clostridium saccharoperbutylacetonicum, or the like. Among these, it is preferable to use Escherichia coli, Saccharomyces cerevisiae, Schizosaccharomyces pombe, or Corynebacterium glutamicum, because transformation thereof is easy, and it is more preferable to use Escherichia coli.

Here, in a manufacturing method for a butanediol according to the present embodiment, it is unnecessary to provide all of genes of enzymes that constitute a biosynthetic pathway that is a target, as exotic genes, by means of transformation, and a gene of an enzyme that is originally held by a host may be utilized to constitute a biosynthetic pathway. Furthermore, although a metabolic pathway originating from a host is utilized to supply acetyl-CoA in the present embodiment, a supply capacity of acetyl-CoA may be improved by a metabolic engineering technique such as gene amplification, introduction of an exotic gene, or knockout of a divergent pathway.

Here, a gene that codes an enzyme that catalyzes each reaction in the present embodiment is not limited to a gene as described previously wherein a specific example thereof has been provided, and a homolog gene thereof may be used. A homolog referred to in the present specification includes an ortholog and a paralog. An ortholog refers to a set of corresponding genes among species generated from a gene of a common ancestor by means of speciation and enzymes obtained from such genes. A paralog refers to a set of corresponding genes among species generated by means of not speciation but gene duplication in an identical species and enzymes obtained from such genes. A homolog refers to genes that have an identity in sequences thereof, regardless of an ortholog or a paralog, and enzymes obtained from such genes.

More specifically, a homolog gene in the present embodiment is a gene that has a base sequence with an identity greater than or equal to 90%, preferably an identity greater than or equal to 95%, and more preferably, a gene that is completely identical to such a gene or wherein one or several bases thereof are deleted, substituted, or added, and an enzyme that is obtained from such a gene.

Furthermore, a homolog gene described previously includes a gene that hybridizes with a gene that has a base sequence complementary with a target gene on a stringent condition. Specifically, it is possible to acquire a gene or an enzyme that is obtained by transformation caused by such a gene, by applying a homology retrieval program (for example, BLAST or FASTA) to a publicly-known data base, or based on an ordinary method such as hybridization or polymerase chain reaction (PCR) on a stringent condition that uses a probe that is composed of at least a portion of an identified gene (DNA that is composed of a base sequence complementary with DNA that is composed of a base sequence of such a gene). Furthermore, it is possible for a person(s) skilled in the art to execute self-design by substituting a base sequence or the like. Here, for a stringent condition referred herein, there is provided, for example, a condition for executing hybridization as described in a non-patent document of Molecular Cloning - A LABORATORY MANUAL THIRD EDITION (Joseph Sambrook, David W. Russell., Cold Spring Harbor Laboratory Press). Specifically, a hybridizing condition is to be retained with a probe in a solution that includes 6 x SSC (a composition of 1 x SSC: 0.15 M of sodium chloride, 0.015 M of sodium citrate, pH: 7.0), 0.5% of SDS, 5 x Denhardt's solution, and 100 mg/mL of herring sperm DNA, at a constant temperature of 65 °C for 8 - 16 hours.

Here, an optical selectivity of a butanediol that is obtained in the present embodiment depends on an optical selectivity of an organic acid-CoA body that is a precursor for obtaining butanediol or an optical selectivity of a selected enzyme in a biosynthetic pathway of such an organic acid-CoA body. That is, a chirality of a reaction pathway for obtaining an organic acid-CoA is selected, and thereby, it is possible to manufacture butanediol that has a desired optical selectivity. For example, (R)-3-hydroxybutyryl-CoA is obtained from acetoacetyl-CoA by using acetoacetyl-CoA reductase (PhaB) originating from a Ralstonia genus (Ralstonia) microbe. Furthermore, for example, (S)-3-hydroxybutyryl-CoA is obtained by using acetoacetyl-CoA reductase (hbd) originating from a Clostridium genus (Clostridium) microbe. Therefore, it is possible to obtain (R)-1,3-butanediol or (S)-1,3-butanediol by selecting these enzymes in a manufacturing method in the present embodiment.

### (A culturing condition)

A gene as described above is introduced into and expressed in a microbial body by means of transformation or the like and such a microbe is cultured, whereby it is possible to obtain a butanediol.

For example, a reaction in the present invention is most conveniently achieved in such a manner that a transformed body is cultured in a nutritive medium such as an LB medium at a temperature of 15 °C - 40 °C, desirably 18 °C - 37 °C for about 24 hours, subsequently implanted to a medium with a normal carbon source, for example, a carbon source that is 0.01 - 50%, desirably 0.1 - 30%, of glucose, and continuously cultured at a similar temperature for about 1 hour - 200 hours, wherein a butanediol is stored in a culture solution in such a process. Furthermore, a carbon source may be added continuously or intermittently depending on consumption of a carbon source that is caused by proliferation of a bacteria or proceeding of a reaction, and in such a case, a concentration of a carbon source in a reaction fluid is not limited to one described previously.

As a medium carbon source for culturing a microbe, it is possible to use, for example, a saccharide such as glucose, sucrose, or fructose, a polyol such as glycerol, an organic substance such as ethanol, acetic acid, citric acid, succinic acid, lactic acid, benzoic acid, or a fatty acid, or an alkali metal salt thereof, an aliphatic hydrocarbon such as an n-paraffin, an aromatic hydrocarbon, or a natural organic substance such as peptone, meat extract, fish extract, soybean flour, or bran, singly or in combination thereof, at a concentration of normally 0.01% - 30%, desirably about 0.1% - 20%.

For a medium nitrogen source for culturing a microbe, it is possible to use, for example, an inorganic nitrogen compound such as ammonium sulfate, ammonium phosphate, sodium nitrate, or potassium nitrate, a nitrogen-containing organic substance such as urea or uric acid, or a natural organic substance such as peptone, meat extract, fish extract, or soybean flour, singly or in combination thereof, at a concentration of normally 0.01% - 20%, desirably about 0.1% - 10%.

Moreover, it is possible to add a phosphate such as potassium dihydrogen phosphate, or a metal salt such as magnesium sulfate, ferrous sulfate, calcium acetate, manganese chloride, copper sulfate, zinc sulfate, cobalt sulfate, or nickel sulfate, as necessary, for growth of a bacteria or improvement of an enzyme activity. A concentration for addition thereof is different depending on a culturing condition, and normally, is 0.01% - 5% for a phosphate, 10 ppm - 1% for a magnesium salt, or about 0.1 ppm - 1,000 ppm for other compounds. Furthermore, for a source of supply for a vitamin, an amino acid, a nucleic acid, or the like, it is possible to add, for example, about 1 ppm - 100 ppm of yeast extract, casamino acid, or a yeast nucleic acid, depending on a selected medium, for growth of a bacteria or improvement of an enzyme activity.

It is desirable to adjust a pH of a medium to 4.5 - 9, desirably 5 - 8. Furthermore, it is useful to fractionate from a culture solution by a method such as centrifugation or membrane filtration, and again suspend and react in water that includes a reaction raw material, physiological saline, a buffer that has a pH adjusted to be comparable to a pH for culturing and composed of phosphoric acid, acetic acid, boric acid, tris(hydroxymethyl)aminomethane, or the like, or a salt thereof, or the like, a microbial or bacterial body that is preliminarily cultured in a medium as described previously, in order to reduce an impurity in a reaction fluid and simplify subsequent fractionation of a product. Although a pH during a reaction is able to be normally retained in a case where a buffer with a sufficient concentration is used, it is desirable to be adjusted appropriately by using sodium hydroxide, ammonia, or the like so as to provide a similar pH in a case where deviation from a pH as described above is caused by proceeding of a reaction.

It is possible to execute separation, recovery, and purification of a butanediol produced in a reaction fluid by using a general means of separation, recovery, and purification of an organic compound, after a bacterial body is eliminated from such a reaction fluid by means of centrifugation at a point of time when an amount of a produced butanediol reaches a substantial amount, or for such a reaction fluid directly. For example, extraction from a filtrate wherein a bacterial body and the others are eliminated from a culture solution is executed by using an appropriate organic solvent. Such an extract is directly distilled away, is further extracted again with an appropriate solvent, purified by using silica gel chromatography or the like, or is subjected to multistage distillation or the like, and thereby, a butanediol is obtained at a high purity.

In a case where a butanediol is stored during a reaction and thereby a reaction rate is lowered, a method is preferable that adds water, physiological saline, a reaction buffer, or the like, into a reaction fluid depending on a concentration of a product to execute continuous dilution thereof. Furthermore, at a point of time when a reaction rate is lowered, a bacteria is fractionated and a supernatant is recovered as a product solution, wherein a fractionated bacteria is again returned to a solution or suspension that includes a reaction raw material and thereby it is possible to recover a reaction rate. It is possible to execute such an operation continuously or even batch-wise by using a centrifuge, a separation film, or the like.

### PRACTICAL EXAMPLES

### (Practical Example 1)

A blunt end fragment of a gene sequence indicated by sequence number 3 (that corresponded to aldehyde reductase) was totally synthesized to add CAT (that corresponded to a restriction enzyme NdeI site in combination with an initiation codon) to a 5'-end and GAGCTC (that corresponded to an SacI site) to a 3'-end by an ordinary method and inserted into an SmaI site in multi-cloning sites of pUC18 to obtain a plasmid. An obtained plasmid was treated with a restriction enzyme to obtain a NdeI - SacI fragment that contained a region of sequence 3. Such a gene fragment was inserted into a NdeI - SacI site in multi-cloning sites of an expression vector pET17b (produced by Novagen, Inc.) by an ordinary method to obtain pETBD3.

A blunt end fragment of a gene sequence indicated by sequence number 1 (that corresponded to catoacetyl-CoA synthase) was totally synthesized by an ordinary method and inserted into an SmaI site in multi-cloning sites of pUC18 to obtain a plasmid. In PCR wherein an obtained plasmid was a template, an amplification fragment was prepared by adding sequences complementary with a front and a back of a NdeI site in multi-cloning sites of pET17b to a front and a back of a region of sequence number 1 and inserted into a NdeI site of pET17b by In-Fusion HD Cloning Kit (produced by TAKARA BIO INC.) to obtain pETBD1 that contained sequence 1.

A gene sequence indicated by sequence number 2 (that corresponded to acetoacetyl-CoA reductase) was inserted into pET17b by a method similar to that of pETBD1 to obtain pETBD2 that contained sequence 2.

An EcoRI site derived from multi-cloning sites of pET17b that was positioned at a downstream of a termination codon of sequence 1 of pETBD3 was cleaved by a restriction enzyme treatment to prepare a ring-opened fragment of pETBD3. Then, a fragment was prepared by PCR in such a manner that sequences that corresponded to 15 bp at an upstream side and 15 bp at a downstream side that contained the EcoRI site of pETBD3 described previously were added upstream and downstream of a region of sequence 1 of pETBD1 and a region that contained a T7 promoter derived from pET17b upstream thereof. Ligation of two obtained fragments was executed by In-Fusion HD Cloning Kit to obtain pETBD31 that contained sequences 3 and 1. Similarly, a PCR fragment that contained a T7 promoter prepared from pETBD2 as a template and sequence 2 was inserted into a ring-opened fragment of pETBD31 that used an XhoI site positioned downstream of sequence 1 of pETBD31, to obtain pETBD312 that contained sequences 3, 1, and 2.

Here, bands with predetermined sizes from a PCR amplification product used in each process were fractionated, excised, and purified by means of electrophoresis and used in a ligation process.

Escherichia coli JM109 (DE3) strain was transformed by using obtained pETBD312 and an ordinary method. A transformed body pETBD312 / JM109 (DE3) was aerobically cultured in 5 mL of an LB medium that contained 100 mg/L of ampicillin at 37 °C for 12 hours. 0.1 mL of a culture solution was implanted into 5 mL of an LB medium that contained 1% of glucose, 100 mg/L of ampicillin, and 0.2 mM of IPTG and aerobically cultured at 30 °C for 48 hours. A supernatant of a culture solution was subjected to high performance liquid chromatography (HPLC: column; Shodex SH-1011 (produced by Showa Denko K. K.), column temperature: 60 °C, eluent: 25 mM sulfuric acid aqueous solution, flow rate 0.6 mL/min, detection: differential refraction detector). In a culture solution, 140 mg/L of 1,3-butanediol was detected.

### (Practical Example 2)

A blunt end fragment of a gene sequence indicated by sequence number 4 (that corresponded to alcohol / aldehyde reductase) was totally synthesized by an ordinary method and inserted into an SmaI site in multi-cloning sites of pUC18 to obtain a plasmid. A blunt end fragment was prepared by PCR in such a manner that 15 bp at each of an upstream side and a downstream side of a part of pETBD312 prepared by a method similar to that of Practical Example 1 which part corresponded to sequence number 3 were added to an obtained plasmid as a template.

A straight-chain blunt fragment was prepared in such a manner that sequence 3 was eliminated from pETBD312, by inverse PCR that used a primer that anneals outward from an upstream side or a downstream side of a part of pETBD312 prepared by a method similar to that of Practical Example 1 which part corresponded to sequence number 3.

Two obtained blunt end fragments were coupled by In-Fusion HD Cloning Kit to prepare pETBD412 in such a manner that sequence 3 of pETBD312 was substituted by sequence 4. Escherichia coli JM109 (DE3) strain was transformed by using obtained pETBD412 and an ordinary method.

Escherichia coli JM109 (DE3) strain was subjected to culturing and analysis in a method similar to that of Practical Example 1. 90 mg/L of 1,3-buatnediol was detected in a culturing solution.

### (Practical Example 3)

A blunt end fragment of a gene sequence indicated by sequence number 5 (that corresponded to a DtsRI subunit of acetyl-CoA carboxylase) was totally synthesized by an ordinary method and inserted into an SmaI site in multi-cloning sites of pUC18 to obtain a plasmid. A sequence that corresponded to each of 15 bp at an upstream side and 15 bp at a downstream side that contained "TA" in an NdeI site of multi-cloning sites of pRSFDuet (produced by Novagen, Inc.) in addition to 15 bp at each of a 5'-end and a 3'-end of sequence 4 was added to an obtained plasmid as a template to prepare a primer with 30 bp at each of both ends, and an amplification reaction was executed to obtain a blunt end fragment that contained sequence 5. Ligation of an obtained blunt end fragment and a straight-chain fragment obtained by applying a restriction enzyme treatment with restriction enzyme NdeI to pRSFDuet was executed by In-Fusion HD Cloning Kit to obtain pRSBD05 that contained sequence 5.

A blunt end fragment of a gene sequence indicated by sequence number 6 (that corresponded to an AccBC subunit of acetyl-CoA carboxylase) was totally synthesized by an ordinary method and inserted into an SmaI site in multi-cloning sites of pUC18 to obtain a plasmid. Sequences that corresponded to an upstream side that contained "CC" and a downstream side that contained "ATGG" in an NdeI site of multi-cloning sites 1 of pRSFDuet in addition to 15 bp at each of a 5'-end and a 3'-end of sequence 6 were added to an obtained plasmid as a template to prepare a primer, and an amplification reaction was executed to obtain a blunt end fragment that contained sequence 6. Ligation of an obtained blunt end fragment and a straight-chain blunt fragment provided by applying inverse PCR to pRSBD05 outward of a restriction enzyme NcoI site was executed by In-Fusion HD Cloning Kit to obtain pRSBD65 that contained sequence 5 and sequence 6.

Escherichia coli JM109 (DE3) strain was transformed by using obtained pRSBD65 and pETBD312 obtained in Practical Example 1 and an ordinary method.

Obtained transformed body pETBD312 + pRSBD65 / JM109 (DE3) was aerobically cultured in 5 mL of an LB medium that contained 100 mg/L of ampicillin and 50 mg/L of kanamycin at 37 °C for 12 hours. 0.1 mL of a culture solution was implanted to 5 mL of an LB medium that contained 1% of glucose, 100 mg/L of ampicillin, 50 mg/L of kanamycin, and 0.2 mM of IPTG and aerobically cultured at 30 °C for 48 hours. A culture solution was subjected to HPLC by a method similar to that of Practical Example 1. 360 mg/L of 1,3-butanediol was detected in a culture solution.

### (Practical Example 4)

A blunt end fragment of a gene sequence indicated by sequence number 8 (that corresponded to an artificially synthesized gene, 4-hydroxybutyryl-CoA dehydratase) was totally synthesized to add CATATG (that corresponded to a NdeI site) to a 5'-end and AAGCTT (that corresponded to a HindIII site) to a 3'-end by an ordinary method and inserted into an SmaI site in multi-cloning sites of pUC18 to obtain a plasmid. An obtained plasmid was treated with a restriction enzyme to obtain a NdeI - HindIII fragment that contained a region of sequence 8. Such a gene fragment was inserted into a NdeI - HindIII site in multi-cloning sites of an expression vector pET17b (produced by Novagen, Inc.) by using an ordinary method to obtain pETSD8. PCR was executed for a primer with such pETSD8 as a template annealed upstream pET17b-vector-derived T7 promoter of pETSD8 and further GGATCC (that corresponded to a BamHI site) added to 5' side and a primer being complementary with 3'-end of sequence 8 of pETSD8 and further provided with GAGCTC (that corresponded to an SacI site) added to 5'-end. An obtained amplified product was treated with SacI to obtain fragment 8.

A blunt end fragment of a gene sequence indicated by sequence number 7 (that corresponded to enoyl-CoA hydratase) was totally synthesized to add CATATG (that corresponded to a NdeI site) to a 5'-end and CTCGAG (that corresponded to a XhoI site) to a 3'-end by an ordinary method and inserted into an SmaI site in multi-cloning sites of pUC18 to obtain a plasmid. An obtained plasmid was treated with a restriction enzyme to obtain an NdeI - XhoI fragment that contained a region of sequence 7. An obtained gene fragment was inserted into an NdeI - XhoI site of multi-cloning sites 2 of expression vector pETDuet (produced by Novagen, Inc.) by an ordinary method to obtain pEDSD07. PCR was executed by using a primer with such pEDSD07 as a template annealed upstream pETDuet-vector-derived T7Lac promoter of pEDSD07 and further GAGCTC (that corresponded to a SacI site) added to 5' side and a primer being complementary with 3'-end of sequence 7 of pEDSD7 and further provided with a complementary sequence of GGATCC (that corresponded to a BamHI site) added to 5'-end. An obtained amplified substance was SacI-treated to obtain fragment 7.

Ligation of fragment 8 and fragment 7 was executed at both SacI site by an ordinary method to provide a serial fragment 87 that contained sequence 8 and sequence 7. After fragment 87 was further amplified by PCR, a BamHI-treated fragment treated with BamHI was obtained. After ligation of an obtained BamHI-treated fragment and a BamHI-treated fragment of pETBD312 was executed by an ordinary method, an insert direction was confirmed by mapping that used PCR and pETBD38712 was obtained such that a translation direction of all inserted sequences are configured in an identical direction.

Escherichia coli Rosetta Blue (DE3) strain (produced by Novagen, Inc.) was transformed by using obtained pETBD38712 and an ordinary method.

Transformed body pETBD38712 / Rosetta Blue (DE3) was aerobically cultured in 5 mL of an LB medium that contained 100 mg/L of ampicillin and 32 mg/L of chloramphenicol at 37 °C for 12 hours. 0.1 mL of a culture solution was implanted to 5 mL of evaluation medium 1 that contained 100 mg/L of ampicillin, 32 mg/L of chloramphenicol, and 0.2 mM of IPTG and aerobically cultured at 30 °C for 48 hours. A detail of evaluation medium 1 was as described below.

A composition of evaluation medium 1 was:
2% glucose;
1% peptone (produced by Difco Inc.);
0.5% yeast extract (produced by Difco Inc.);
2.4% K₂HPO₄;
0.6% KH₂PO₄;
600 ppm iron citrate; and
100 ppm riboflavin / distilled water (pH 7.2).

After all of composition substances described above were mixed and stirred at ordinary temperature for 2 hours, filtration sterilization was executed by using a filter with 0.45 µm.

A culture solution after culturing was subjected to HPLC by a method similar to that of Practical Example 1. 220 mg/L of 1,3-butanediol and 18 mg/L of 1,4-butanediol were detected in a culture solution.

### (Practical Example 5)

Escherichia coli Rosetta Blue (DE3) strain was transformed by using pRSBD65 obtained in Practical Example 3 and pETBD38712 obtained in Practical Example 4 and an ordinary method.

Obtained transformed body pRSBD65 + pETBD38712 / Rosetta Blue (DE3) was aerobically cultured in 5 mL of an LB medium that contained 100 mg/L of ampicillin, 50 mg/L of kanamycin, and 32 mg/L of chloramphenicol at 37 °C for 12 hours.

0.1 mL of a culture solution was implanted to 5 mL of evaluation medium 1 that contained 100 mg/L of ampicillin, 50 mg/L of kanamycin, 32 mg/L of chloramphenicol, and 0.2 mM of IPTG and aerobically cultured at 30 °C for 48 hours.

A culture solution was subjected to HPLC by a method similar to that of Practical Example 1. 410 mg/L of 1,3-butanediol and 50 mg/L of 1,4-butanediol were detected in a culture solution.

The present application claims priority based on Japanese Patent Application No. 2012-209981 filed on September 24, 2012 before the Japan Patent Office and the entire contents of Japanese Patent Application No. 2012-209981 are incorporated by reference in the present application.

## Claims

1. A manufacturing method for a butanediol, including a step that cultures a microbe that contains a gene that codes acetoacetyl-CoA synthase that catalyzes a reaction that irreversibly produces acetoacetyl-CoA from acetyl-CoA and malonyl-CoA, acetoacetyl-CoA reductase that catalyzes a reaction that produces 3-hydroxybutyryl-CoA from acetoacetyl-CoA, and an enzyme that catalyzes a reaction for producing a butanediol from 3-hydroxybutyryl-CoA.

2. The manufacturing method for a butanediol as claimed in claim 1, wherein the enzyme that catalyzes a reaction for producing a butanediol from 3-hydroxybutyryl-CoA is enoyl-CoA hydratase that catalyzes a reaction that produces crotonyl-CoA from 3-hydroxybutyryl-CoA, 4-hydroxybutyryl-CoA dehydratase that catalyzes a reaction that produces 4-hydroxybutyryl-CoA from crotonyl-CoA, and an aldehyde dehydrogenase that catalyzes a reaction that produces 4-hydroxybutanal from 4-hydroxybutyryl-CoA or an alcohol dehydrogenase that catalyzes a reaction that produces 1,4-butanediol therefrom.

3. The manufacturing method for a butanediol as claimed in claim 1, wherein the enzyme that catalyzes a reaction for producing a butanediol from 3-hydroxybutyryl-CoA is an aldehyde dehydrogenase that catalyzes a reaction that produces 3-hydroxybutanal from 3-hydroxybutyryl-CoA or an alcohol dehydrogenase that catalyzes a reaction that produces 1,3-butanediol therefrom.

4. The manufacturing method for a butanediol as claimed in claim 1, wherein a gene that codes acetoacetyl-CoA synthase that catalyzes a reaction that irreversibly produces acetoacetyl-CoA from acetyl-CoA and malonyl-CoA is a gene described in any of undermentioned (a) - (c) :
(a) a gene that has a base sequence of sequence number 1;
(b) a gene that has a base sequence such that one or more bases are deleted, substituted, or added in a base sequence of sequence number 1, wherein the gene has a base sequence with an identity greater than or equal to 90% with respect to the base sequence of sequence number 1;
(c) a gene that hybridizes with a gene that has a base sequence complimentary with a gene that has a base sequence described in sequence number 1 on a stringent condition.

5. The manufacturing method for a butanediol as claimed in claim 1, wherein the microbe contains a gene that codes 4-hydroxybutyryl-CoA dehydratase described in any of undermentioned (d) - (f):
(d) a gene that has a base sequence of sequence number 8;
(e) a gene that has a base sequence such that one or more bases are deleted, substituted, or added in a base sequence of sequence number 8, wherein the gene has a base sequence with an identity greater than or equal to 90% with respect to the base sequence of sequence number 8;
(f) a gene that hybridizes with a gene that has a base sequence complimentary with a gene that has a base sequence described in sequence number 8 on a stringent condition.

6. The manufacturing method for a butanediol as claimed in claim 1, wherein the microbe further contains a gene that codes acetyl-CoA carboxylase that catalyzes a reaction that produces malonyl-CoA while acetyl-CoA is a substrate.

7. The manufacturing method for a butanediol as claimed in claim 1, wherein the step that cultures includes a step that cultures the microbe on an aerobic condition.

8. The manufacturing method for a butanediol as claimed in claim 1, wherein the microbe is Escherichia coli, a yeast, a corynebacterium, or a clostridium.


SEQUENCE LISTING

<110>  Showa Denko K.K.
<120>  Method of producing butanediols
<130>  P20120132/SHD13006PCT
<160>  8
<170>  PatentIn version 3.5

<210>  1
<211>  990
<212>  DNA
<213>  Artificial Sequence
<220>
<223>  Streptomyces sp. nphT7 gene
<400>  1
atgaccgacg tccgattccg cattatcggt acgggtgcct acgtaccgga acggatcgtc
60

tccaacgatg aagtcggcgc gccggccggg gtggacgacg actggatcac ccgcaagacc
120

ggtatccggc agcgtcgctg ggccgccgac gaccaggcca cctcggacct ggccacggcc
180

gcggggcggg cagcgctgaa agcggcgggc atcacgcccg agcagctgac cgtgatcgcg
240

gtcgccacct ccacgccgga ccggccgcag ccgcccacgg cggcctatgt ccagcaccac
300

ctcggtgcga ccggcactgc ggcgttcgac gtcaacgcgg tctgctccgg caccgtgttc
360

gcgctgtcct cggtggcggg caccctcgtg taccggggcg gttacgcgct ggtcatcggc
420

gcggacctgt actcgcgcat cctcaacccg gccgaccgca agacggtcgt gctgttcggg
480

gacggcgccg gcgcaatggt cctcgggccg acctcgaccg gcacgggccc catcgtccgg
540

cgcgtcgccc tgcacacctt cggcggcctc accgacctga tccgtgtgcc cgcgggcggc
600

agccgccagc cgctggacac ggatggcctc gacgcgggac tgcagtactt cgcgatggac
660

gggcgtgagg tgcgccgctt cgtcacggag cacctgccgc agctgatcaa gggcttcctg
720

cacgaggccg gggtcgacgc cgccgacatc agccacttcg tgccgcatca ggccaacggt
780

gtcatgctcg acgaggtctt cggcgagctg catctgccgc gggcgaccat gcaccggacg
840

gtcgagacct acggcaacac gggagcggcc tccatcccga tcaccatgga cgcggccgtg
900

cgcgccggtt ccttccggcc gggcgagctg gtcctgctgg ccgggttcgg cggcggcatg
960

gccgcgagct tcgccctgat cgagtggtag
\201@\201@\201@\201@\201@\201@\201@\201@\201@\201@\201@\201@     990

<210>  2
<211>  741
<212>  DNA
<213>  Artificial Sequence
<220>
<223>  Ralstonia eutropha phaB gene
<400>  2
atgactcagc gcattgcgta tgtgaccggc ggcatgggtg gtatcggaac cgccatttgc
60

cagcggctgg ccaaggatgg ctttcgtgtg gtggccggtt gcggccccaa ctcgccgcgc
120

cgcgaaaagt ggctggagca gcagaaggcc ctgggcttcg atttcattgc ctcggaaggc
180

aatgtggctg actgggactc gaccaagacc gcattcgaca aggtcaagtc cgaggtcggc
240

gaggttgatg tgctgatcaa caacgccggt atcacccgcg acgtggtgtt ccgcaagatg
300

acccgcgccg actgggatgc ggtgatcgac accaacctga cctcgctgtt caacgtcacc
360

aagcaggtga tcgacggcat ggccgaccgt ggctggggcc gcatcgtcaa catctcgtcg
420

gtgaacgggc agaagggcca gttcggccag accaactact ccaccgccaa ggccggcctg
480

catggcttca ccatggcact ggcgcaggaa gtggcgacca agggcgtgac cgtcaacacg
540

gtctctccgg gctatatcgc caccgacatg gtcaaggcga tccgccagga cgtgctcgac
600

aagatcgtcg cgacgatccc ggtcaagcgc ctgggcctgc cggaagagat cgcctcgatc
660

tgcgcctggt tgtcgtcgga ggagtccggt ttctcgaccg gcgccgactt ctcgctcaac
720

ggcggcctgc atatgggctg a
\201@\201@\201@\201@\201@\201@\201@\201@\201@\201@\201@\201@          741

<210>  3
<211>  1407
<212>  DNA
<213>  Artificial Sequence
<220>
<223>  Clostridium klyuveri ald gene
<400>  3
atgaataaag acacactaat acctacaact aaagatttaa aagtaaaaac aaatggtgaa
60

aacattaatt taaagaacta caaggataat tcttcatgtt tcggagtatt cgaaaatgtt
120

gaaaatgcta taagcagcgc tgtacacgca caaaagatat tatcccttca ttatacaaaa
180

gagcaaagag aaaaaatcat aactgagata agaaaggccg cattacaaaa taaagaggtc
240

ttggctacaa tgattctaga agaaacacat atgggaagat atgaggataa aatattaaaa
300

catgaattgg tagctaaata tactcctggt acagaagatt taactactac tgcttggtca
360

ggtgataatg gtcttacagt tgtagaaatg tctccatatg gtgttatagg tgcaataact
420

ccttctacga atccaactga aactgtaata tgtaatagca taggcatgat agctgctgga
480

aatgctgtag tatttaacgg acacccatgc gctaaaaaat gtgttgcctt tgctgttgaa
540

atgataaata aggcaattat ttcatgtggc ggtcctgaaa atctagtaac aactataaaa
600

aatccaacta tggagtctct agatgcaatt attaagcatc cttcaataaa acttctttgc
660

ggaactgggg gtccaggaat ggtaaaaacc ctcttaaatt ctggtaagaa agctataggt
720

gctggtgctg gaaatccacc agttattgta gatgatactg ctgatataga aaaggctggt
780

aggagcatca ttgaaggctg ttcttttgat aataatttac cttgtattgc agaaaaagaa
840

gtatttgttt ttgagaatgt tgcagatgat ttaatatcta acatgctaaa aaataatgct
900

gtaattataa atgaagatca agtatcaaaa ttaatagatt tagtattaca aaaaaataat
960

gaaactcaag aatactttat aaacaaaaaa tgggtaggaa aagatgcaaa attattctta
1020

gatgaaatag atgttgagtc tccttcaaat gttaaatgca taatctgcga agtaaatgca
1080

aatcatccat ttgttatgac agaactcatg atgccaatat tgccaattgt aagagttaaa
1140

gatatagatg aagctattaa atatgcaaag atagcagaac aaaatagaaa acatagtgcc
1200

tatatttatt ctaaaaatat agacaaccta aatagatttg aaagagaaat agatactact
1260

atttttgtaa agaatgctaa atcttttgct ggtgttggtt atgaagcaga aggatttaca
1320

actttcacta ttgctggatc tactggtgag ggaataacct ctgcaaggaa ttttacaaga
1380

caaagaagat gtgtacttgc cggctaa
\201@\201@\201@\201@\201@\201@\201@\201@\201@\201@\201@\201@        1407

<210>  4
<211>  2577
<212>  DNA
<213>  Artificial Sequence
<220>
<223>  Clostridium acetobutylicum adhE2 gene
<400>  4
atgaaagtta caaatcaaaa agaactaaaa caaaagctaa atgaattgag agaagcgcaa
60

aagaagtttg caacctatac tcaagagcaa gttgataaaa tttttaaaca atgtgccata
120

gccgcagcta aagaaagaat aaacttagct aaattagcag tagaagaaac aggaataggt
180

cttgtagaag ataaaattat aaaaaatcat tttgcagcag aatatatata caataaatat
240

aaaaatgaaa aaacttgtgg cataatagac catgacgatt ctttaggcat aacaaaggtt
300

gctgaaccaa ttggaattgt tgcagccata gttcctacta ctaatccaac ttccacagca
360

attttcaaat cattaatttc tttaaaaaca agaaacgcaa tattcttttc accacatcca
420

cgtgcaaaaa aatctacaat tgctgcagca aaattaattt tagatgcagc tgttaaagca
480

ggagcaccta aaaatataat aggctggata gatgagccat caatagaact ttctcaagat
540

ttgatgagtg aagctgatat aatattagca acaggaggtc cttcaatggt taaagcggcc
600

tattcatctg gaaaacctgc aattggtgtt ggagcaggaa atacaccagc aataatagat
660

gagagtgcag atatagatat ggcagtaagc tccataattt tatcaaagac ttatgacaat
720

ggagtaatat gcgcttctga acaatcaata ttagttatga attcaatata cgaaaaagtt
780

aaagaggaat ttgtaaaacg aggatcatat atactcaatc aaaatgaaat agctaaaata
840

aaagaaacta tgtttaaaaa tggagctatt aatgctgaca tagttggaaa atctgcttat
900

ataattgcta aaatggcagg aattgaagtt cctcaaacta caaagatact tataggcgaa
960

gtacaatctg ttgaaaaaag cgagctgttc tcacatgaaa aactatcacc agtacttgca
1020

atgtataaag ttaaggattt tgatgaagct ctaaaaaagg cacaaaggct aatagaatta
1080

ggtggaagtg gacacacgtc atctttatat atagattcac aaaacaataa ggataaagtt
1140

aaagaatttg gattagcaat gaaaacttca aggacattta ttaacatgcc ttcttcacag
1200

ggagcaagcg gagatttata caattttgcg atagcaccat catttactct tggatgcggc
1260

acttggggag gaaactctgt atcgcaaaat gtagagccta aacatttatt aaatattaaa
1320

agtgttgctg aaagaaggga aaatatgctt tggtttaaag tgccacaaaa aatatatttt
1380

aaatatggat gtcttagatt tgcattaaaa gaattaaaag atatgaataa gaaaagagcc
1440

tttatagtaa cagataaaga tctttttaaa cttggatatg ttaataaaat aacaaaggta
1500

ctagatgaga tagatattaa atacagtata tttacagata ttaaatctga tccaactatt
1560

gattcagtaa aaaaaggtgc taaagaaatg cttaactttg aacctgatac tataatctct
1620

attggtggtg gatcgccaat ggatgcagca aaggttatgc acttgttata tgaatatcca
1680

gaagcagaaa ttgaaaatct agctataaac tttatggata taagaaagag aatatgcaat
1740

ttccctaaat taggtacaaa ggcgatttca gtagctattc ctacaactgc tggtaccggt
1800

tcagaggcaa caccttttgc agttataact aatgatgaaa caggaatgaa atacccttta
1860

acttcttatg aattgacccc aaacatggca ataatagata ctgaattaat gttaaatatg
1920

cctagaaaat taacagcagc aactggaata gatgcattag ttcatgctat agaagcatat
1980

gtttcggtta tggctacgga ttatactgat gaattagcct taagagcaat aaaaatgata
2040

tttaaatatt tgcctagagc ctataaaaat gggactaacg acattgaagc aagagaaaaa
2100

atggcacatg cctctaatat tgcggggatg gcatttgcaa atgctttctt aggtgtatgc
2160

cattcaatgg ctcataaact tggggcaatg catcacgttc cacatggaat tgcttgtgct
2220

gtattaatag aagaagttat taaatataac gctacagact gtccaacaaa gcaaacagca
2280

ttccctcaat ataaatctcc taatgctaag agaaaatatg ctgaaattgc agagtatttg
2340

aatttaaagg gtactagcga taccgaaaag gtaacagcct taatagaagc tatttcaaag
2400

ttaaagatag atttgagtat tccacaaaat ataagtgccg ctggaataaa taaaaaagat
2460

ttttataata cgctagataa aatgtcagag cttgcttttg atgaccaatg tacaacagct
2520

aatcctaggt atccacttat aagtgaactt aaggatatct atataaaatc attttaa
2577

<210> 5
<211> 1632
<212> DNA
<213> Artificial Sequence
<220>
<223> Corynebacterium glutamicum dtsR1_ACC gene
<400> 5
atgaccattt cctcaccttt gattgacgtc gccaaccttc cagacatcaa caccactgcc
60

ggcaagatcg ccgaccttaa ggctcgccgc gcggaagccc atttccccat gggtgaaaag
120

gcagtagaga aggtccacgc tgctggacgc ctcactgccc gtgagcgctt ggattactta
180

ctcgatgagg gctccttcat cgagaccgat cagctggctc gccaccgcac caccgctttc
240

ggcctgggcg ctaagcgtcc tgcaaccgac ggcatcgtga ccggctgggg caccattgat
300

ggacgcgaag tctgcatctt ctcgcaggac ggcaccgtat tcggtggcgc gcttggtgag
360

gtgtacggcg aaaagatgat caagatcatg gagctggcaa tcgacaccgg ccgcccattg
420

atcggtcttt acgaaggcgc tggcgctcgt attcaggacg gcgctgtctc cctggacttc
480

atttcccaga ccttctacca aaacattcag gcttctggcg ttatcccaca gatctccgtc
540

atcatgggcg catgtgcagg tggcaacgct tacggcccag ctctgaccga cttcgtggtc
600

atggtggaca agacctccaa gatgttcgtt accggcccag acgtgatcaa gaccgtcacc
660

ggcgaggaaa tcacccagga agagcttggc ggagcaacca cccacatggt gaccgctggt
720

aactcccact acaccgctgc gaccgatgag gaagcactgg attgggtaca ggacctggtg
780

tccttcctcc catccaacaa tcgctcctac gcaccgatgg aagacttcga cgaggaagaa

840

ggcggcgttg aagaaaacat caccgctgac gatctgaagc tcgacgagat catcccagat
900

tccgcgaccg ttccttacga cgtccgcgat gtcatcgaat gcctcaccga cgatggcgaa
960

tacctggaaa tccaggcaga ccgcgcagaa aacgttgtta ttgcattcgg ccgcatcgaa
1020

ggccagtccg ttggctttgt tgccaaccag ccaacccagt tcgctggctg cctggacatc
1080

gactcctctg agaaggcagc tcgcttcgtc cgcacctgcg acgcgttcaa catcccaatc
1140

gtcatgcttg tcgacgtccc cggcttcctc ccaggcgcag gccaggagta cggtggcatt
1200

ctgcgtcgtg gcgcaaagct gctctacgca tacggcgaag caaccgttcc aaagatcacc
1260

gtcaccatgc gtaaggctta cggcggagcg tactgcgtga tgggttccaa gggcttgggc
1320

tctgacatca accttgcatg gccaaccgca cagatcgccg tcatgggcgc tgctggcgca
1380

gttggattca tctaccgcaa ggagctcatg gcagctgatg ccaagggcct cgataccgta
1440

gctctggcta agtccttcga gcgcgagtat gaagaccaca tgctcaaccc gtaccacgct
1500

gcagaacgtg gcctgatcga cgccgtgatc ctgccaagcg aaacccgcgg acagatttcc
1560

cgcaaccttc gcctgctcaa gcacaagaac gtcactcgcc ctgctcgcaa gcacggcaac
1620

atgccactgt aa
\201@\201@\201@\201@\201@\201@\201@\201@\201@\201@\201@\201@
1632



<210>  6
<211>  1776
<212>  DNA
<213>  Artificial Sequence
<220>
<223>  Corynebacterium glutamicum accBC_ACC gene
<400>  6
atgtcagtcg agactaggaa gatcaccaag gttcttgtcg ctaaccgtgg tgagattgca
60

atccgcgtgt tccgtgcagc tcgagatgaa ggcatcggat ctgtcgccgt ctacgcagag
120

ccagatgcag atgcaccatt cgtgtcatat gcagacgagg cttttgccct cggtggccaa
180

acatccgctg agtcctacct tgtcattgac aagatcatcg atgcggcccg caagtccggc

240

gccgacgcca tccaccccgg ctacggcttc ctcgcagaaa acgctgactt cgcagaagca
300

gtcatcaacg aaggcctgat ctggattgga ccttcacctg agtccatccg ctccctcggc
360

gacaaggtca ccgctcgcca catcgcagat accgccaagg ctccaatggc tcctggcacc
420

aaggaaccag taaaagacgc agcagaagtt gtggctttcg ctgaagaatt cggtctccca
480

atcgccatca aggcagcttt cggtggcggc ggacgtggca tgaaggttgc ctacaagatg
540

gaagaagtcg ctgacctctt cgagtccgca acccgtgaag caaccgcagc gttcggccgc
600

ggcgagtgct tcgtggagcg ctacctggac aaggcacgcc acgttgaggc tcaggtcatc
660

gccgataagc acggcaacgt tgttgtcgcc ggaacccgtg actgctccct gcagcgccgt
720

ttccagaagc tcgtcgaaga agcaccagca ccattcctca ccgatgacca gcgcgagcgt
780

ctccactcct ccgcgaaggc tatctgtaag gaagctggct actacggtgc aggcaccgtt
840

gagtacctcg ttggctccga cggcctgatc tccttcctcg aggtcaacac ccgcctccag
900

gtggaacacc cagtcaccga agagaccacc ggcatcgacc tggtccgcga aatgttccgc
960

atcgcagaag gccacgagct ctccatcaag gaagatccag ctccacgcgg ccacgcattc
1020

gagttccgca tcaacggcga agacgctggc tccaacttca tgcctgcacc aggcaagatc
1080

accagctacc gcgagccaca gggcccaggc gtccgcatgg actccggtgt cgttgaaggt
1140

tccgaaatct ccggacagtt cgactccatg ctggcaaagc tgatcgtttg gggcgacacc
1200

cgcgagcagg ctctccagcg ctcccgccgt gcacttgcag agtacgttgt cgagggcatg
1260

ccaaccgtta tcccattcca ccagcacatc gtggaaaacc cagcattcgt gggcaacgac
1320

gaaggcttcg agatctacac caagtggatc gaagaggttt gggataaccc aatcgcacct
1380

tacgttgacg cttccgagct cgacgaagat gaggacaaga ccccagcaca gaaggttgtt
1440

gtggagatca acggccgtcg cgttgaggtt gcactcccag gcgatctggc actcggtggc

1500

accgctggtc ctaagaagaa ggccaagaag cgtcgcgcag gtggtgcaaa ggctggcgta
1560

tccggcgatg cagtggcagc tccaatgcag ggcactgtca tcaaggtcaa cgtcgaagaa
1620

ggcgctgaag tcaacgaagg cgacaccgtt gttgtcctcg aggctatgaa gatggaaaac
1680

cctgtgaagg ctcataagtc cggaaccgta accggcctta ctgtcgctgc aggcgagggt
1740

gtcaacaagg gcgttgttct cctcgagatc aagtaa
\201@\201@\201@\201@\201@\201@\201@\201@\201@\201@  1776


<210>  7
<211>  405
<212>  DNA
<213>  Artificial Sequence
<220>
<223>  Aeromonas caviae phaJ gene
<400>  7
atgagcgcac aatccctgga agtaggccag aaggcccgtc tcagcaagcg gttcggggcg
60

gcggaggtag ccgccttcgc cgcgctctcg gaggacttca accccctgca cctggacccg
120

gccttcgccg ccaccacggc gttcgagcgg cccatagtcc acggcatgct gctcgccagc
180

ctcttctccg ggctgctggg ccagcagttg ccgggcaagg ggagcatcta tctgggtcaa
240

agcctcagct tcaagctgcc ggtctttgtc ggggacgagg tgacggccga ggtggaggtg
300

accgcccttc gcgaggacaa gcccatcgcc accctgacca cccgcatctt cacccaaggc
360

ggcgccctcg ccgtgacggg ggaagccgtg gtcaagctgc cttaa
\201@\201@\201@\201@    405


<210>  8
<211>  1473
<212>  DNA
<213>  Artificial Sequence
<220>
<223>  putative 4-hydroxybutyryl-CoA dehydratase gene
<220>
<223>  Inventor: Aoki, Hirobumi\201G Kokido, Yuzuru; Yoneda, Tadashi
<400>  8
atgctgatga ccgccgaaca gtacatcgaa agcctgcgca agctgaacac gcgtgtctac
60

atgtttggtg aaaagatcga gaactgggta gaccatccca tgattcgccc ttcgatcaat
120

tgtgtggcaa tgacctatga actggcgcaa gatccccagt atgccgattt gatgaccacg

180

aaatcgaatc tgattggcaa gacgattaat cgcttcgcga acttacacca aagcaccgat
240

gatctgcgca agaaagtcaa aatgcaacgt ctgctgggcc agaaaaccgc gagttgcttt
300

cagcggtgtg ttgggatgga tgcgttcaat gccgtgtttt ccaccactta tgagattgat
360

cagaaatacg ggactaacta ccacaagaac ttcacggagt atctgaaata tattcaagaa
420

aatgacttga tcgtggatgg tgctatgacc gacccgaaag gcgaccgtgg gctggcaccg
480

agtgcccaga aagacccaga tctgtttctg cgcatcgtgg agaaacgcga ggatggtatc
540

gtagtccgtg gtgcgaaagc acatcaaacg ggctcaatta atagccatga gcacatcatt
600

atgccgacca ttgctatgac agaagcggat aaagactatg cggttagttt tgcttgtcca
660

tccgatgcgg atggtttgtt tatgatctat ggtcgccagt cttgcgacac ccgcaaaatg
720

gaggaaggcg cagatattga cttaggcaac aaacagtttg gcggccaaga agcccttgtc
780

gtgtttgaca acgtattcat cccgaatgat cgcattttct tatgccagga atacgatttt
840

gcgggaatga tggtcgaacg cttcgctggt tatcatcgcc agtcttacgg tggctgcaaa
900

gtgggtgtgg gtgacgtcgt tattggagcc gcagcccttg cagcggatta caacggtgcc
960

cagaaggcca gccatgtgaa agacaaactg atcgaaatga cacacttgaa cgaaaccctg
1020

tattgttgtg ggattgcgtg ctcagccgaa ggttatccga ctgcagccgg caattaccag
1080

atcgatctcc tccttgcgaa tgtgtgcaaa cagaacatta cgcgttttcc gtacgagatt
1140

gtacgtctgg cggaagatat tgccggagga ctgatggtta ctatgcctag cgaagcggac
1200

ttcaaaagcg aaacggttgt tgggcgtgat ggcgaaacca ttggcgattt ctgcaacaaa
1260

ttctttgcgg cagcaccgac ttgcaccaca gaagaacgga tgcgtgtgct ccgctttctg
1320

gagaacattt gtttaggcgc atccgctgtt gggtatcgta cagaatcgat gcatggtgcg
1380

ggctctccac aggctcaacg catcatgatt gcccgtcaag gcaatattaa cgcgaagaaa

1440

gaactcgcta aagcgatcgc tggcatcaaa taa
\201@\201@\201@\201@\201@\201@\201@\201@\201@\201@\201@\201@ 1473